# FASCICULE DE BREVET EUROPEEN

(11) **EP 1 895 917 B1**
(45) Date de publication et mention de la délivrance du brevet: **17.09.2008**
(21) Numéro de dépôt: 05778796.2
(22) Date de dépôt: 22.06.2005
(51) Int. Cl.: A61B 17/30

(54) **PINCE CHIRURGICALE DE MICROCHIRURGIE OPHTALMOLOGIQUE, À USAGE UNIQUE**
OPHTHALMOLOGISCHE MIKROCHIRURGISCHE ZANGE ZUR EINMALIGEN VERWENDUNG
OPHTHALMOLOGIC MICROSURGICAL FORCEPS FOR SINGLE USE

(43) Date de publication de la demande: 12.03.2008
(73) Titulaire: André, Jean-Marie, 13001 Marseille (FR)
(72) Inventeur: André, Jean-Marie, 13001 Marseille (FR)
(74) Mandataire: Marek, Pierre
(86) Numéro de dépôt international: PCT/FR2005/001569
(87) Numéro de publication internationale: WO 2006/136663

(56) Documents cités:
- EP-A- 0 159 453
- WO-A-98/22035
- GB-A- 2 239 417
- US-A- 3 677 112
- US-A- 4 041 952
- US-A- 4 452 106
- US-A- 4 727 876
- US-A- 4 793 349
- US-A- 6 106 542

## Description

La présente invention concerne une pince de microchirurgie ophtalmologique, à usage unique, plus spécialement dédiée à la chirurgie de la cataracte.

Plus précisément, l'invention est relative à une pince de microchirurgie oculaire jetable du genre comportant deux branches reliées l'une à l'autre par l'une de leurs extrémités pour former la partie proximale de préhension de la pince et dont les extrémités opposées sont constituées par des pointes de pincement susceptibles d'être rapprochées élastiquement l'une de l'autre et constituant la partie distale active de ladite pince.

L'opération de la cataracte est actuellement l'intervention chirurgicale la plus pratiquée dans le monde. Cette intervention nécessite une série d'instruments et notamment des pinces de microchirurgie du genre susmentionné, comportant des pointes de pincement extrêmement fines qui, en cours d'utilisation, doivent pouvoir être amenées rigoureusement en regard l'une de l'autre.

Actuellement, ces instruments de grande précision sont le plus souvent fabriqués en un métal dur et inoxydable, de manière artisanale et manuelle, ce qui les rend très coûteux.

De telles pinces sont, par exemple, décrites dans les documents FR-2.644.689 et FR-2.491.325, ce dernier exposant les raisons du caractère obligatoirement très onéreux de ces instruments.

Le coût de fabrication élevé est un obstacle majeur s'opposant à ce que ces instruments soient proposés comme instruments "jetables", alors que leur non réutilisation serait sanitairement et économiquement souhaitable. En effet, la stérilisation et/ou la décontamination obligatoire des instruments chirurgicaux réutilisables et leur conservation à l'état aseptisé, entre deux utilisations successives, imposent d'importants investissements en personnel qualifié, en matériel et en temps de travail. D'autre part, ces instruments deviennent inutilisables dès que leurs pointes sont quelque peu déformées ou émoussées et ne présentent plus la précision de fonctionnement indispensable.

Pour abaisser le prix de revient de ces instruments de sorte à en faire des articles à usage unique, il faut parvenir à en mécaniser la production.

Dans le document WO-03/04509, est décrite une pince chirurgicale jetable entièrement réalisée par moulage sous pression d'une matière thermoplastique et se présentant sous forme d'une seule pièce monobloc.

L'idée de réaliser les pinces de microchirurgie oculaire en matière plastique thermoformée peut paraître d'emblée très séduisante, en raison du caractère économique de ce mode de production. Cependant, les praticiens ont pu constater que si ce matériau convient pour la fabrication des manches ou parties proximales des branches des instruments, il ne présente pas les caractéristiques physiques indispensables de finesse et de dureté propres au métal, dans la partie distale active (pointes de pincement des branches) desdits instruments.

Pour remédier aux inconvénients susmentionnés des instruments chirurgicaux entièrement réalisés en métal ou des instruments chirurgicaux complètement exécutés en matière plastique, on a déjà proposé de réaliser certains de ces instruments avec une partie active métallique surmoulée par une matière plastique injectée sous pression (par exemple : US-2003/0109858, EP-1.262.157, WO-98/22035, US-3.677.112, US-4.041.952).

Le document WO-98/22035 décrit un genre de pince chirurgicale jetable correspondant au-preambule de la première revendication et comprenant deux branches munies de pointes métalliques constituées d'inserts surmoulés.

Grâce à un tel mode de fabrication, il est possible de réaliser des instruments chirurgicaux de façon plus économique permettant de les réserver à un usage unique.

Cependant, la souplesse des matières plastiques qu'il est possible d'utiliser pour la fabrication des pinces de microchirurgie oculaire soulève des problèmes que la présente invention se propose de solutionner.

Le principal de ces problèmes réside dans la constatation du fait que si, pour les pinces entièrement métalliques, la rigidité du métal autorise un parfait alignement des extrémités distales constituées par les pointes de pincement des branches des pinces, lors de leur rapprochement, sans qu'il soit besoin de mettre en place un centreur particulier, la souplesse des manches ou parties proximales desdites branches, lorsqu'ils sont réalisés en matière plastique, ne permet pas de garantir un tel alignement parfait en cours d'utilisation.

Par exemple, dans le document WO-98/22035, aucune disposition permettant un alignement rigoureux des extrémités de pincement n'est prévue, de sorte que la pince de micro-chirurgie décrite dans ce document n'est pas utilisable pour des interventions de microchirurgie ophtalmologique.

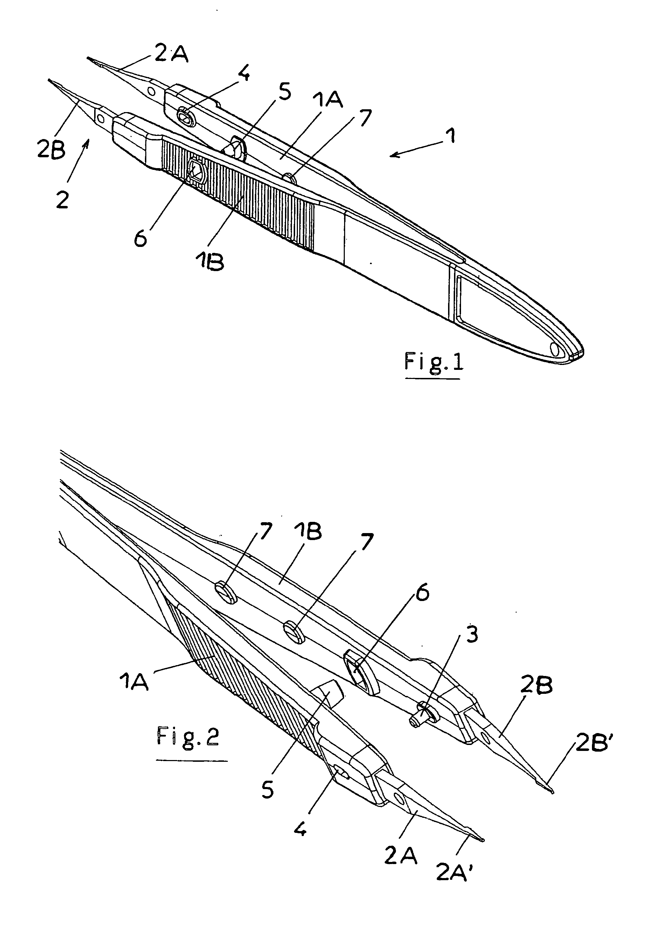

D'autre part, le document US-4.041.952 décrit une pince pour électrocoagulation dont la destination et la fonction ne sont donc pas les mêmes que celles de la pince de microchirurgie ophtalmologique utilisable pour des interventions sous microscope opératoire selon l'invention. Par ailleurs, selon les modes d'exécution illustrés, la partie de préhension de la pince n'est pas moulée d'une seule pièce en matière plastique. Selon le mode d'exécution de la figure 2, la partie de préhension de la pince comporte deux bras séparés par une pièce d'écartement et maintenus ensemble par un manchon : il y a donc quatre pièces moulées séparément ; tandis que, selon le mode d'exécution de la figure 15, la partie de préhension de la pince comprend une partie en forme de fourche obtenue par une première opération de moulage et comportant deux bras munis de gorges dans lesquelles sont positionnés les composants électriques de la pince qui sont ensuite enfermés dans lesdites gorges par une deuxième opération de moulage. Dans le premier cas, la fixation non simultanée des inserts métalliques d'électrocoagulation par un procédé de rivetage ne garantit pas un positionnement réciproque rigoureux desdits inserts, ni celui des moyens de centrage. Dans le second cas, ce positionnement rigoureux n'est pas davantage garanti en raison de la deuxième opération de moulage qui peut perturber le positionnement obtenu par la première.

Dans le document US-3.677.112, est décrite une pince pour la manipulation de petits objets fragiles tels que des composants électroniques. Une telle pince est inutilisable pour des interventions de micro-chirurgie ophtalmologique. En effet, chaque pointe de pincement est d'abord insérée dans l'une des extrémités d'une branche et les deux branches sont ensuite assemblées par rivetage à leur extrémité opposée. Un tel procédé ne permet pas de garantir la précision dans le rapprochement des extrémités des pointes de pincement, précision indispensable dans le domaine de la microchirurgie oculaire. En outre, l'absence de moyens de guidage durant le mouvement de rapprochement des pointes de pincement, constitue un empêchement supplémentaire à l'obtention de la grande précision requise dans le domaine particulier de l'invention.

Selon l'invention, le problème exposé ci-dessus est résolu par une pince de microchirurgie ophtalmologique à usage unique, du genre comprenant, d'une part, une partie de préhension moulée d'une seule pièce en matière plastique et constituée de deux bras reliés l'un à l'autre par l'une de leurs extrémités pour former l'extrémité proximale de la pince et, d'autre part, une partie de pincement constituée par des pointes métalliques insérées lors du moulage de ladite partie de préhension, dans les extrémités distales desdits bras, de sorte à former les deux branches de ladite pince, la partie distale active de cette pince étant munie de moyens de centrage comprenant, d'une part, une partie mâle, de préférence constituée par un ergot ou doigt de forme cylindrique solidaire de l'une des branches de la pince et orienté en direction de l'autre branche, et, d'autre part, une partie femelle constituée par un orifice transfixiant ménagé dans ladite autre branche, en regard de ladite partie mâle, ces moyens de centrage étant prévus dans les portions d'extrémité des bras en matière plastique dans lesquelles se trouvent implantées les portions proximales des pointes de pincement métalliques, de sorte que ledit orifice de centrage transfixie ou traverse non seulement la matière plastique mais également la partie de la pointe métallique enchâssée dans ladite matière plastique.

Grâce à cette disposition, les extrémités actives des pointes de pincement métalliques de la pince peuvent être rapprochées jusqu'à se trouver au contact l'une de l'autre, avec une grande précision.

L'orifice transfixiant à la fois la matière plastique et la partie métallique permet de situer les moyens de centrage le plus bas possible sur les branches de la pince et donc le plus près possible des pointes de pincement de cette dernière.

D'autre part, la prévision de cet orifice transfixiant permet d'obtenir les deux bras de la pince en une seule opération de moulage.

Selon un mode de réalisation préféré, la pince est aussi pourvue de moyens de pré-centrage comprenant, d'une part, une partie mâle, par exemple constituée par un tenon solidaire de l'une des branches de la pince et orienté en direction de l'autre branche et, d'autre part, une partie femelle constituée par un orifice transfixiant que présente l'autre branche, en regard de ladite partie mâle.

Ces moyens de pré-centrage sont disposés à faible distance (par exemple à une distance de l'ordre de 11,1 mm) des moyens de centrage précédemment décrits, dans la direction de l'extrémité proximale de la pince.

De manière avantageuse, le tenon et l'orifice transfixiant de pré-centrage présentent une forme oblongue dont le grand axe est orienté perpendiculairement à l'axe de la branche.

Le tenon et l'orifice de précentrage présentent un volume plus important que celui de l'ergot et de l'orifice de centrage.

Lors du rapprochement des branches de la pince, les moyens de pré-centrage entrent les premiers en action, ce qui permet un affrontement suffisant desdites branches pour que les moyens de centrage puissent ensuite parfaitement remplir leur fonction.

Un autre problème rencontré par l'utilisation de pinces chirurgicales composites du genre susmentionné, du fait de la souplesse de la matière plastique constituant les bras de la pince, est qu'il arrive que, lorsque les extrémités actives des pointes métalliques se trouvent pressées l'une contre l'autre, la pression exercée sur lesdits bras par les doigts du praticien peut être plus importante que nécessaire, ce qui a pour conséquence de créer un effet de levier sur un point d'appui situé entre la zone de pression des doigts et lesdites extrémités actives (par exemple constitué par le centreur, voire même par la plate-forme métallique desdites pointes) entraînant un léger écartement de ces dernières, c'est-à-dire un résultat exactement opposé à l'effet de pincement recherché.

Selon une autre disposition caractéristique de l'invention, on résoud ce problème par la prévision, sur la face interne de l'un au moins des deux bras en matière plastique et au moins dans la zone de préhension de la pince, d'une ou de plusieurs butées orientées en direction de la face interne de l'autre bras.

Selon un mode d'exécution préféré, des butées coopérantes sont disposées en regard les unes des autres sur la face interne de chaque manche en matière plastique de la pince.

Ces butées s'opposent efficacement à un rapprochement non souhaitable des manches de la pince au delà de leur mouvement permettant aux pointes de pincement de ladite pince de venir au contact l'une de l'autre, même en cas d'une pression excessive appliquée par les doigts du praticien, en cours d'opération. D'autre part, la prévision d'une pluralité de butées coopérantes espacées réparties dans la zone de préhension de la pince contribue au bon positionnement réciproque des bras de cette dernière, dans la position de pincement.

Les buts, caractéristiques et avantages ci-dessus, et d'autres encore, ressortiront mieux de la description qui suit et des dessins annexés dans lesquels:
La figure 1 est une vue en perspective d'un exemple de réalisation d'une pince de microchirurgie oculaire exécutée selon l'invention.
La figure 2 est une vue en perspective et à plus grande échelle de la partie distale de cette pince.

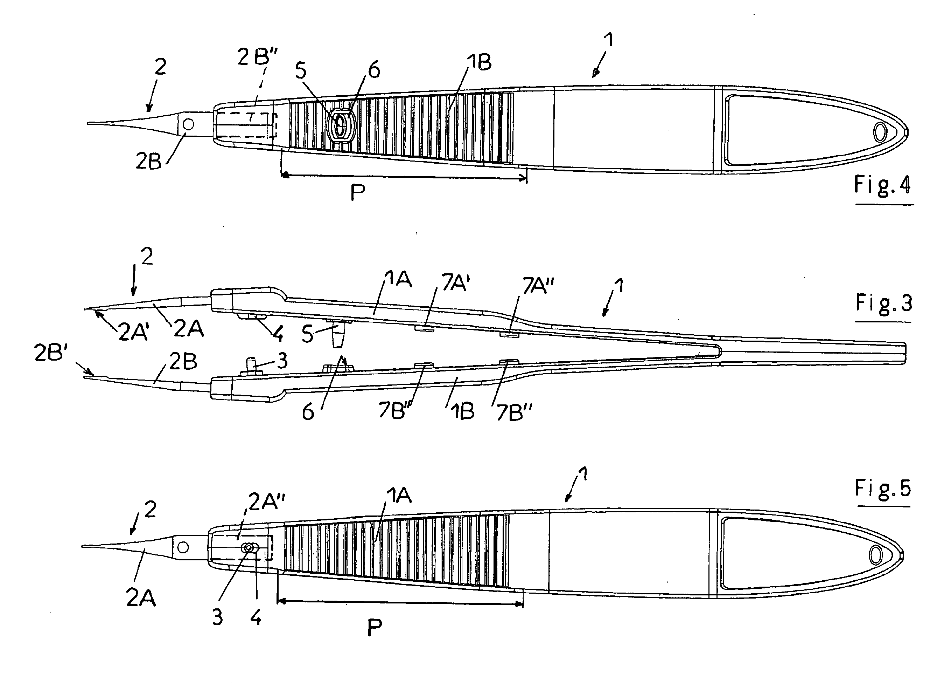

La figure 3 est une vue en élévation et de face de la pince.
Les figures 4 et 5 sont des vues de côté montrant les faces latérales opposées de la pince.

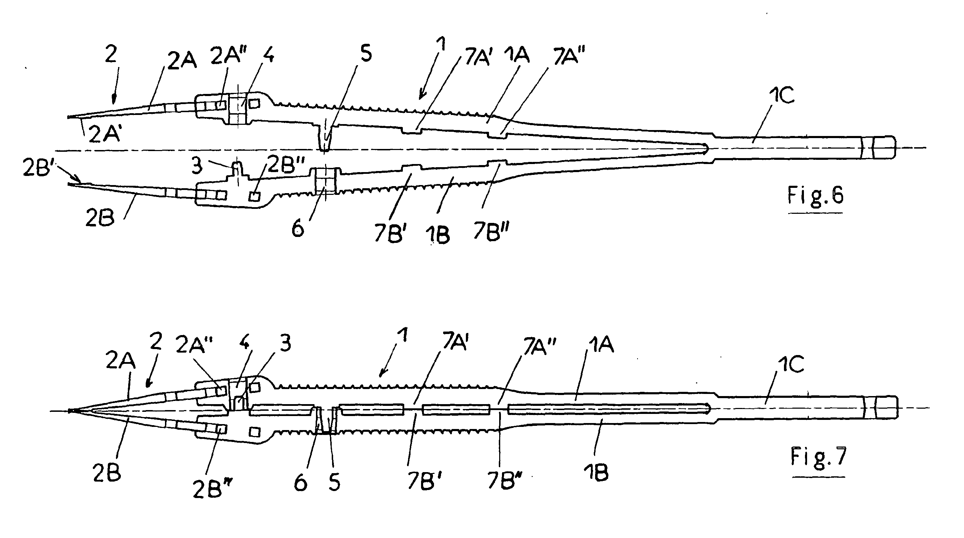

La figure 6 est une vue en coupe longitudinale de la pince représentée en position de repos.
La figure 7 est une vue en coupe longitudinale montrant le rapprochement des pointes actives de la pince, en position de pincement.
Les figures 8 et 9 sont des vues en coupe, et à échelle agrandie, selon les lignes 8-8 et 9-9, respectivement, de la figure 3.
Les figures 10 et 11 sont des vues partielles montrant les faces internes des parties distales des deux branches de la pince.

On se réfère auxdits dessins pour décrire un exemple de réalisation avantageux, quoique non limitatif, d'une pince de microchirurgie ophtalmologique à usage unique, selon l'invention qui, selon cet exemple est constituée par l'une des pinces de microchirurgie oculaire utilisable dans le processus opératoire de la cataracte.

La pince à laquelle se réfère l'invention est du genre comportant deux branches reliées l'une à l'autre, par l'une de leurs extrémités pour former la partie proximale de la pince et dont les extrémités opposées sont constituées par des pointes de pincement susceptibles d'être rapprochées élastiquement l'une de l'autre et constituant la partie distale active de ladite pince.

La pince selon l'invention est une pince composite dont la partie de préhension manuelle est exécutée en matière plastique rigide et flexible telle que, par exemple, Zylar (Marque Déposée), Terpolimères (ABS), Polycarbonates (PC), et dont la partie de pincement est réalisée en un métal dur et inaltérable tel qu'acier inoxydable titane ou autre.

Cette pince composite est exécutée en une seule pièce, en surmoulant ses parties de pincement métalliques par toute matière plastique présentant les caractéristiques de dureté et de flexibilité requises, par tout procédé d'injection sous pression convenable.

De la sorte, la fabrication des pointes métalliques de pincement dans un métal présentant la dureté souhaitable peut être mécanisée et automatisée, et les pinces peuvent être obtenues en une seule opération d'injection, ce qui permet une production très économique de ces dernières et leur réservation à un usage unique.

Les pinces ainsi réalisées comprennent : d'une part, une partie de préhension 1 moulée d'une seule pièce en matière plastique et constituée de deux bras 1A, 1B reliés l'un à l'autre par l'une de leur extrémité pour constituer l'extrémité proximale de la pince et s'étendant à partir de leur jonction en formant un angle aigu, par exemple de l'ordre de 6° à 7°, et, d'autre part, une partie de pinçage 2 constituée par des pointes métalliques 2A, 2B implantées par surmoulage, lors du moulage de ladite partie de préhension, dans les extrémités distales desdits bras.

Les bras 1A, 1 B peuvent être rapprochés élastiquement l'un de l'autre en raison de la flexibilité de la matière dans laquelle ils sont exécutés, de sorte à amener les extrémités actives 2A', 2B' des pointes de pincement 2A, 2B, respectivement, au contact l'une de l'autre.

La partie distale de la pince ainsi réalisée est munie de moyens de centrage permettant un guidage des extrémités actives 2A', 2B' des pointes de pincement 2A, 2B lors de leur déplacement l'une vers l'autre.

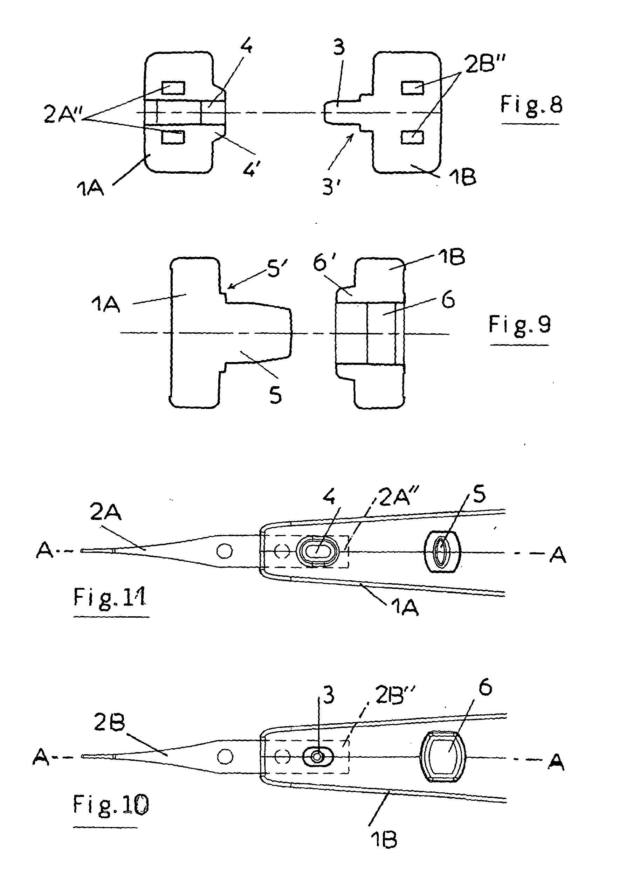

Selon une première disposition caractéristique de l'invention, ces moyens de centrage comprennent, d'une part, une partie mâle, de préférence constituée par un pivot 3 de forme cylindrique formé d'une seule pièce avec l'un 1 B des bras en matière plastique de la pince et orienté en direction de l'autre bras 1A de celle-ci et, d'autre part, une partie femelle, constituée par un orifice transfixiant 4 de forme oblongue, ménagée dans le bras 1A, en regard dudit ergot ou doigt 3. Cet orifice présente une forme oblongue dont le grand axe est parallèle à l'axe A-A du bras 1A muni dudit orifice (figure 11).

Le pivot 3 et l'orifice 4 sont disposés dans les portions d'extrémité des bras en matière plastique dans lesquelles se trouvent noyées les portions proximales des pointes de pincement métalliques 2B et 2A, respectivement, de sorte que ledit orifice de centrage 4 transfixie ou traverse non seulement la matière plastique mais également la partie 2A" de la pointe métallique de pincement 2A insérée dans ladite matière plastique.

Selon une autre disposition caractéristique très avantageuse de l'invention, la pince est aussi pourvue de moyens de pré-centrage comprenant, d'une part, une partie mâle, de préférence constituée par un tenon 5 formé d'une seule pièce avec l'un des bras de la pince et orienté en direction de l'autre bras et, d'autre part, une partie femelle constituée par un orifice transfixiant 6 que présente l'autre bras, en regard de ladite partie mâle.

Ces moyens de pré-centrage 5, 6 sont disposés à faible distance des moyens de centrage 3, 4, dans la direction de l'extrémité de jonction des bras 1A, 1B de la pince.

Selon un mode d'exécution préféré, le tenon 5 et l'orifice transfixiant 6 de pré-centrage présentent une forme oblongue, par exemple approximativement ovale, dont le grand axe est orienté perpendiculairement aux axes A-A et A'-A' des bras 1A et 1B munis dudit tenon et dudit orifice, respectivement (figure 10). D'autre part, le tenon 5 et l'orifice 6 de pré-centrage présentent des volumes plus importants que ceux du pivot 3 et de l'orifice 4 de centrage, respectivement.

La disposition des éléments constitutifs mâles et femelles des moyens de centrage 3, 4 et des moyens de pré-centrage 5, 6 sur les bras 1A et 1B est de préférence inversée, c'est-à-dire que le pivot de centrage 3 est prévu sur le bras 1 B alors que le tenon de pré-centrage 5 est disposé sur le bras 1A.

Selon une autre disposition caractéristique de l'invention, la face interne de l'un au moins des bras 1A, 1 B réalisés en matière plastique, est munie, au moins dans la zone de préhension ou de pression digitale P desdits bras, d'une ou, de préférence, plusieurs butées 7, orientée en direction de la face interne de l'autre bras 1B, 1A, de sorte à limiter l'amplitude du mouvement nécessaire desdits bras pour amener les extrémités actives 2A', 2B' des pointes de pincement au contact l'une de l'autre.

Par exemple, la face interne de l'un au moins des bras peut être pourvue de deux butées espacées 7 constituées par des ergots cylindriques orientés en direction de la face interne de l'autre bras. Alternativement, la face interne de l'un au moins des bras pourrait être munie d'une unique butée de grande longueur couvrant par exemple la majeure partie de la longueur de la zone de préhension de ces derniers.

Selon un mode d'exécution préféré, des butées coopérantes 7A', 7B' et 7A", 7B" sont disposées en regard les unes des autres sur la face interne de chaque bras en matière plastique 1A, 1 B, entre les moyens de centrage 5 ou 6 et les extrémités de jonction desdits bras.

Ces butées sont dimensionnées de sorte que lorsqu'une force de rapprochement est appliquée dans la zone de préhension P des bras 1A, 1 B, les butées 7A', 7A" du bras 1A, sont pressées contre les butées 7B', 7B" du bras 1B, dès que les extrémités actives 2A', 2B' des pointes de contact entrent au contact l'une de l'autre.

On comprend que grâce à ce système de butée, aucune pression excessive susceptible d'entraîner un décentrage des extrémités actives 2A', 2B', en cours d'utilisation ne peut être communiquée à celles-ci.

De manière intéressante, comme le montrent les figures 8 et 9, les orifices de centrage 4 et/ou de pré-centrage 6 sont bordés par une collerette, respectivement 4' et 6', faisant saillie sur la face interne des bras 1A et 1B.

D'autre part, le doigt de centrage 3 et/ou l'ergot de pré-centrage 5 comportent, à leur base, un épaulement circulaire 3' et 5', respectivement.

Ces collerettes 4' et 6' et ces épaulements circulaires 3' et 5' remplissent une fonction comparable à celle des butées 7A', 7B', 7A" et 7B", lorsque l'ergot de pré-centrage 5 et le doigt de centrage 3 sont engagés dans les orifices de pré-centrage 6 et de centrage 4.

Lors du rapprochement des branches 1A-2A et 1B-2B de la pince, les butées 7A', 7A", l'épaulement 5' et la collerette 4' du bras 1A entrent en contact avec les butées 7B', 7B", la collerette 6' et l'épaulement 3' du bras 1B, respectivement, ce qui stoppe le mouvement desdites branches l'une vers l'autre dans la position de serrage souhaitable des extrémités actives 2A', 2B' des pointes de pincement 2A, 2B en supprimant toute possibilité de pincement trop énergique quelle que soit la force de la pression appliquée par le praticien.

Les différentes butées 7A'-7B', 7A"-7B", 5'-6', 3'-4', sont réparties sur toute la longueur de la zone de préhension de la pince, de sorte qu'il n'existe aucune possibilité de basculement de l'une des branches autour d'un point fixe d'appui, en cas de pression trop importante sur lesdites branches.

## Revendications

1. Pince de microchirurgie ophtalmologique à usage unique, du genre comprenant, d'une part, une partie de préhension (1) moulée d'une seule pièce en matière plastique et constituée de deux bras (1A, 1B) reliés l'un à l'autre par l'une de leurs extrémités pour former l'extrémité proximale de la pince et, d'autre part, une partie de pincement (2) constituée par des pointes métalliques (2A, 2B) insérées lors du moulage de ladite partie de préhension, dans les extrémités, distales desdits bras, de sorte à former les deux branches de ladite pince, characterisée et ce que la partie distale de cette pince est munie de moyens de centrage comprenant, d'une part, une partie mâle, de préférence constituée par un ergot ou doigt (3) de forme cylindrique solidaire de l'une des branches de la pince et orienté en direction de l'autre branche, et, d'autre part, une partie femelle constituée par un orifice transfixiant (4) ménagé dans ladite autre branche, en regard de ladite partie mâle, ces moyens de centrage étant prévus dans les portions d'extrémité des bras en matière plastique (1A, 1 B) dans lesquelles se trouvent insérées les portions proximales (2A", 2B") des pointes de pincement métalliques, de sorte que ledit orifice de centrage (4) transfixie ou traverse non seulement la matière plastique mais également la partie (2A") de la pointe métallique (2A) enchâssée dans ladite matière plastique.

2. Pince de microchirurgie ophtalmologique, à usage unique, selon la revendication 1, **caractérisée en ce que** l'orifice transfixiant (4) présente une forme oblongue dont le grand axe est parallèle à l'axe (A-A) du bras (1A) muni dudit orifice (4).

3. Pince de microchirurgie ophtalmologique, à usage unique, selon l'une des revendications 1 ou 2, **caractérisée en ce qu'**elle est munie de moyens de pré-centrage comprenant, d'une part, une partie mâle, par exemple constituée par un tenon (5) solidaire de l'une des branches de la pince et orienté en direction de l'autre branche et, d'autre part, une partie femelle constituée par un orifice transfixiant (6) que présente l'autre branche, en regard de ladite partie mâle, ces moyens de pré-centrage (5,6) étant disposés à distance des moyens de centrage (3,4), dans la direction de l'extrémité proximale de la pince.

4. Pince de microchirurgie ophtalmologique, à usage unique, suivant la revendication 3, **caractérisée en ce que** la disposition des éléments constitutifs mâles et femelles des moyens de centrage (3,4) et des moyens de pré-centrage (5,6) sur les branches (1A-2A, 1B-2B) de la pince, est inversée.

5. Pince de microchirurgie ophtalmologique, à usage unique, selon l'une des revendications 3 ou 4, **caractérisée en ce que** le tenon (5) et l'orifice transfixiant (6) de pré-centrage présentent une forme oblongue, par exemple une forme approximativement ovale, dont le grand axe est orienté perpendiculairement aux axes (A-A, A'-A') des bras (1A, 1B) munis dudit tenon et dudit orifice, respectivement.

6. Pince de microchirurgie ophtalmologique, à usage unique, selon l'une quelconque des revendications 3 à 5, **caractérisée en ce que** le tenon (5) et l'orifice (6) de pré-centrage présentent des volumes plus importants que ceux du pivot (3) et de l'orifice de centrage (4), respectivement.

7. Pince de microchirurgie ophtalmologique, à usage unique, selon l'une quelconque des revendications 1 à 6, **caractérisée en ce que** la face interne de l'un au moins des bras en matière plastique (1A, 1B) est munie, au moins dans la zone de préhension ou de pression digitale (P) desdits bras, d'une ou plusieurs butées (7) orientée(s) en direction de la face interne de l'autre bras (1 B, 1A).

8. Pince de microchirurgie ophtalmologique, à usage unique, selon la revendication 7, **caractérisée en ce que** la face interne de chaque bras en matière plastique (1A, 1B) est munie de butées coopérantes espacées (7A', 7B' et 7A", 7B") disposées en regard les unes des autres, entre les moyens de pré-centrage (5,6) et les extrémités de jonction desdits bras.

9. Pince de microchirurgie ophtalmologique, à usage unique, selon l'une des revendications 7 ou 8, **caractérisée en ce que** le doigt de centrage (3) et/ou l'ergot de pré-centrage (5) comportent, à leur base, un épaulement circulaire (3', 5'), et **en ce que** les orifices de centrage (4) et/ou de pré-centrage (6) sont bordés par une collerette (4', 6'), ces épaulements et ces collerettes venant au contact les uns des autres dans la position de pincement, de sorte à constituer des butées lors du rapprochement des branches de la pince.

10. Pince de microchirurgie ophtalmologique, à usage unique, selon l'une quelconque des revendications 7 à 9, **caractérisée en ce que** les butées (7A'-7B', 7A"-7B", 5'-6', 3'-4') limitant le mouvement de rapprochement des bras (1A, 1 B), sont réparties sur toute la longueur de la zone de préhension (P) de la pince.

## Claims

1. Single-use ophthalmological microsurgery forceps, of the kind comprising on the one hand a gripping part (1) moulded in one piece of plastic material and formed by two arms (1A, 1B) connected together at one of their ends to form the proximal end of the forceps and on the other hand a clamping part (2) formed by metal tips (2A, 2B) which are inserted upon moulding of said gripping part into the distal ends of said arms so as to form the two limbs of said forceps, **characterised in that** the distal part of said forceps is provided with centering means comprising on the one hand a male part preferably formed by a pin or finger (3) of cylindrical shape fixed with respect to one of the limbs of the forceps and oriented in the direction of the other limb and on the other hand a female part formed by a through opening (4) provided in said other limb in facing relationship with said male part, said centering means being provided in the end portions of the arms (1A, 1B) of plastic material in which the proximal portions (2A", 2B") of the metal clamping tips are inserted, in such a way that said centering opening (4) transfixes or passes through not only the plastic material but also the part (2A") of the metal tip (2A) mounted in said plastic material.

2. Single-use ophthalmological microsurgery forceps according to claim 1 **characterised in that** the through opening (4) is of an oblong shape, the long axis of which is parallel to the axis (A-A) of the arm (1A) provided with said opening (4).

3. Single-use ophthalmological microsurgery forceps according to one of claims 1 and 2 **characterised in that** it is provided with pre-centering means comprising on the one hand a male part for example formed by a projection (5) fixed with respect to one of the limbs of the forceps and oriented in the direction of the other limb and on the other hand a female part formed by a through opening (6) that the other limb has in facing relationship with said male part, said pre-centering means (5, 6) being disposed at a spacing from the centering means (3, 4) in the direction of the proximal end of the forceps.

4. Single-use ophthalmological microsurgery forceps according to claim 3 **characterised in that** the arrangement of the male and female constituent elements of the centering means (3, 4) and the pre-centering means (5, 6) on the limbs (1A-2A, 1B-2B) of the forceps is reversed.

5. Single-use ophthalmological microsurgery forceps according to one of claims 3 and 4 **characterised in that** the pre-centering projection (5) and the through opening (6) are of an oblong shape, for example an approximately oval shape, the long axis of which is oriented perpendicularly to the axes (A-A, A'-A') of the arms (1A, 1B) provided with said projection and said opening respectively.

6. Single-use ophthalmological microsurgery forceps according to any one of claims 3 to 5 **characterised in that** the pre-centering projection (5) and the opening (6) are of larger volumes than those of the pivot (3) and the centering opening (4) respectively.

7. Single-use ophthalmological microsurgery forceps according to any one of claims 1 to 6 **characterised in that** the inside face of one at least of the arms (1A, 1B) of plastic material is provided at least in the gripping or finger pressure zone (P) of said arms with one or more abutments (7) oriented in the direction of the inside face of the other arm (1B, 1A).

8. Single-use ophthalmological microsurgery forceps according to claim 7 **characterised in that** the inside face of each arm (1A, 1B) of plastic material is provided with spaced co-operating abutments (7A', 7B' and 7A", 7B") disposed in mutually facing relationship between the pre-centering means (5, 6) and the junction ends of said arms.

9. Single-use ophthalmological microsurgery forceps according to one of claims 7 and 8 **characterised in that** the centering finger (3) and/or the pre-centering pin (5) comprise at their base a circular shoulder (3', 5') and that the centering (4) and/or pre-centering (6) openings are bordered by a collar (4', 6'), said shoulders and said collars coming into contact with each other in the clamping position in such a way as to constitute abutments when the limbs of the forceps move towards each other.

10. Single-use ophthalmological microsurgery forceps according to any one of claims 7 to 9 **characterised in that** the abutments (7A'-7B', 7A"-7B", 5'-6', 3'-4') limiting the movement of the arms (1A, 1B) towards each other are distributed over the entire length of the gripping zone (P) of the forceps.

## Patentansprüche

1. Ophthalmologische mikrochirurgische Pinzette zur einmaligen Verwendung der Art, die einerseits einen aus einem Stück aus Kunststoff geformten Griffteil (1), der aus zwei miteinander an einem ihrer Enden verbundenen Armen (1A, 1B) besteht, die das proximale Ende der Pinzette bilden, und andererseits einen Greifteil (2) umfasst, der aus Metallspitzen (2A, 2B) besteht, die während des Formens des Griffteils in die distalen Enden der Arme eingesetzt werden, so dass die zwei Schenkel der Pinzette gebildet werden, **dadurch gekennzeichnet, dass** der distale Teil dieser Pinzette mit Mitteln zum Zentrieren ausgestattet ist, die einerseits einen männlichen Teil, der vorzugsweise aus einem Zapfen oder Finger (3) mit zylindrischer Form besteht, der fest mit einem der Schenkel der Pinzette verbunden und in Richtung auf den anderen Schenkel hin ausgerichtet ist, und andererseits einen weiblichen Teil umfassen, der aus einer Durchgangsöffnung (4) besteht, die in dem anderen Schenkel in Bezug auf den männlichen Teil ausgespart ist, wobei diese Mittel zum Zentrieren in den Endabschnitten der Kunststoffarme (1A, 1B) vorgesehen sind, in denen sich die proximalen Abschnitte (2A", 2B") der Metall-Greifspitzen eingesetzt befinden, so dass die Zentrieröffnung (4) nicht nur den Kunststoff durchbohrt oder durchquert, sondern auch den Teil (2A") der Metallspitze (2A), der in den Kunststoff eingebettet ist.

2. Ophthalmologische mikrochirurgische Pinzette zur einmaligen Verwendung nach Anspruch 1, **dadurch gekennzeichnet, dass** die Durchgangsöffnung (4) eine längliche Form aufweist, deren große Achse parallel zu der Achse (A-A) des Arms (1A) ist, der mit der Öffnung (4) versehen ist.

3. Ophthalmologische mikrochirurgische Pinzette zur einmaligen Verwendung nach einem der Ansprüche 1 oder 2, **dadurch gekennzeichnet, dass** sie mit Mitteln zum Vorzentrieren ausgestattet ist, die einerseits einen männlichen Teil, der beispielsweise aus einem Zapfen (5) besteht, der fest mit einem der Schenkel der Pinzette verbunden ist und in Richtung auf den anderen Schenkel hin ausgerichtet ist, und andererseits einen weiblichen Teil umfassen, der aus einer Durchgangsöffnung (6) besteht, die der andere Schenkel in Bezug auf den männlichen Teil aufweist, wobei diese Mittel zum Vorzentrieren (5, 6) zu den Mitteln zum Zentrieren (3, 4) beabstandet in der Richtung des proximalen Endes der Pinzette angeordnet sind.

4. Ophthalmologische mikrochirurgische Pinzette zur einmaligen Verwendung nach Anspruch 3, **dadurch gekennzeichnet, dass** die Anordnung der männlichen und weiblichen Bestandselemente der Mittel zum Zentrieren (3, 4) und der Mittel zum Vorzentrieren (5, 6) auf den Schenkeln (1A-2A, 1B-2B) der Pinzette umgekehrt ist.

5. Ophthalmologische mikrochirurgische Pinzette zur einmaligen Verwendung nach einem der Ansprüche 3 oder 4, **dadurch gekennzeichnet, dass** der Zapfen (5) und die Durchgangsöffnung (6) zum Vorzentrieren eine längliche Form aufweisen, beispielsweise eine annähernd ovale Form, deren große Achse senkrecht zu den Achsen (A-A, A'-A') der Arme (1A, 1B) ausgerichtet ist, die jeweils mit dem Zapfen und der Öffnung versehen sind.

6. Ophthalmologische mikrochirurgische Pinzette zur einmaligen Verwendung nach einem der Ansprüche 3 bis 5, **dadurch gekennzeichnet, dass** der Zapfen (5) und die Öffnung (6) zum Vorzentrieren größere Volumen als die des Zapfens (3) bzw. der Zentrieröffnung (4) aufweisen.

7. Ophthalmologische mikrochirurgische Pinzette zur einmaligen Verwendung nach einem der Ansprüche 1 bis 6, **dadurch gekennzeichnet, dass** die Innenseite wenigstens eines der Kunststoffarme (1A, 1B) wenigstens in dem Griff- oder Fingerdruck-Bereich (P) der Arme mit einem oder mehreren Anschlägen (7) versehen ist, der bzw. die in Richtung auf die Innenseite des anderen Arms (1B, 1A) hin ausgerichtet ist oder sind.

8. Ophthalmologische mikrochirurgische Pinzette zur einmaligen Verwendung nach Anspruch 7, **dadurch gekennzeichnet, dass** die Innenseite jedes Kunststoffarms (1A, 1B) mit zusammenwirkenden beabstandeten Anschlägen (7A', 7B' und 7A", 7B") ausgestattet ist, die in Bezug zueinander zwischen den Mitteln zum Vorzentrieren (5, 6) und den Verbindungsenden der Arme angeordnet sind.

9. Ophthalmologische mikrochirurgische Pinzette zur einmaligen Verwendung nach einem der Ansprüche 7 oder 8, **dadurch gekennzeichnet, dass** der Zentrierfinger (3) und/oder der Vorzentrierzapfen (5) an ihrer Basis einen kreisförmigen Vorsprung (3', 5') aufweisen, und **dadurch**, dass die Öffnungen zum Zentrieren (4) und/oder Vorzentrieren (6) von einem Kragen (4', 6') eingefasst sind, wobei diese Vorsprünge und diese Kragen in der Einklemmposition miteinander in Berührung kommen, so dass sie bei der Annäherung der Schenkel der Pinzette Anschläge bilden.

10. Ophthalmologische mikrochirurgische Pinzette zur einmaligen Verwendung nach einem der Ansprüche 7 bis 9, **dadurch gekennzeichnet, dass** die Anschläge (7A'-7B', 7A"-7B", 5'-6', 3'-4'), die die Annäherungsbewegung der Arme (1A, 1B) begrenzen, auf die gesamte Länge des Greifbereichs (P) der Pinzette verteilt sind.
